# EUROPEAN PATENT SPECIFICATION

(11) **EP 2 977 012 B1**
(45) Date of publication and mention of the grant of the patent: **03.10.2018**
(21) Application number: 15153428.6
(22) Date of filing: 02.02.2015
(51) Int. Cl.: A61B 8/14, A61B 8/08, A61B 8/00

(54) **Ultrasound imaging apparatus and controlling method thereof**
Ultraschallbildgebungsvorrichtung und Steuerverfahren dafür
Appareil d'imagerie ultraacoustique et son procédé de commande

(30) Priority: 24.07.2014 KR 20140094214
(43) Date of publication of application: 27.01.2016
(73) Proprietor: Samsung Medison Co., Ltd., Gangwon-do 250-870 (KR)
(72) Inventor: Jin, Gil Ju, Seoul (KR); Ahn, Mi Jeoung, Seoul (KR); Byun, Mi Ae, Gyeonggi-do (KR)
(74) Representative: Frey, Sven Holger

(56) References cited:
- EP-A1- 2 754 396
- US-A- 5 938 607
- US-A1- 2010 191 114
- US-A1- 2012 220 873
- US-A1- 2012 310 092

## Description

### BACKGROUND

### 1. Field

Embodiments of the present disclosure relate to an ultrasound imaging apparatus for processing an ultrasonic image and a controlling method thereof.

### 2. Description of Related Art.

An ultrasound imaging apparatus irradiates ultrasounds to a target part in an object through the surface of the object, detects echo ultrasounds reflected from the object and then noninvasively provides images about an examined part, such as a tomogram of a soft tissue or bloodstream.

The ultrasound imaging apparatus is compact, inexpensive, and displaying a diagnostic image immediately as compared with another type of diagnostic image apparatus, e.g., X-ray device, Computerized Tomography (CT) scanner, Magnetic Resonance Image (MRI), diagnostic nuclear medical apparatus. In addition, the ultrasound imaging apparatus is safe because there is not risk of radiation exposure. Therefore, the ultrasound imaging apparatus is widely used in medical examination at maternity, cardiology, abdomen, urology clinics.

In general, a diagnostic image generated by an ultrasound imaging apparatus displays a portion of an object, thus it is difficult for an operator to recognize intuitionally a target part of the object or a ultrasound probe direction toward an examined position. Together with the diagnostic image, an image indicator indicating a target part of the object may be displayed, wherein the image indicator may be letters or icons similar with a shape of the object.

The US 2012/310092 A1 describes an ultrasound diagnostic apparatus which includes an input device configured to receive an input to display a cross-section of a reference medical image, wherein the reference medical image identifies a location of the cross-section in relation to a subject, and further includes a characteristic region, and a reference medical image display control device configured to display the cross-section of the reference medical image based on volume data of the reference medical image previously acquired, the cross-section displayed according to the input.

In the US 2012/220873 A1 embodiments of an ultrasound system for outputting an ultrasound image through an ultrasound examination are disclosed. In one embodiment, an ultrasound system comprises an ultrasound probe, a sensor unit, a processor unit and an input unit. The ultrasound probe transmits and receives an ultrasound beam to and from the examination portion of a target object. The sensor unit is disposed in the ultrasound probe and detects a posture and/or a position of the ultrasound probe to form posture information and/or position information of the ultrasound probe. The processor unit moves an image indicator corresponding to the posture information and/or the position information. The image indicator includes an ultrasound beam direction marker that is indicative of a transmission direction of the ultrasound beam transmitted from the ultrasound probe to the target object. The input unit is configured to control the processor unit.

In the EP 2 754 396 A1 an ultrasound image is displayed while clarifying a positional relationship with an ultrasound probe. An ultrasound diagnostic device is provided with an ultrasound probe, a first position detecting means for detecting a position of the ultrasound probe, an ultrasound image generating means for generating an ultrasound image by using a reflected echo signal, an ultrasound volume data generating means for generating three-dimensional ultrasound volume data by accumulating the ultrasound images, a reference image generating means for generating a ultrasound reference image of an arbitrary cross section by using the ultrasound volume data and displaying an ultrasound probe mark indicating the position of the ultrasound probe in a superimposed manner on a position in the ultrasound reference image, the position corresponding to the ultrasound probe position detected by the first position detecting means, and a display means for displaying the ultrasound image and the ultrasound reference image.

In the US 5,938,607 A an ultrasonic diagnostic imaging system is provided which aids in the diagnosis of patient conditions by providing access from the ultrasound system to a library of reference ultrasonic images. The image library is cataloged in accordance with an image characteristic such as the type of examination, the part of the body or the type of pathology shown in the image, and the images of the library are accessed in accordance with these characteristics. The image library may be remotely located and accessible by a number of ultrasound systems over a network, or it may be located on the ultrasound system itself such as on a system disk drive. In a preferred embodiment reference images are concurrently displayed with patient images to aid in discerning the patient's condition.

### SUMMARY

Therefore, it is an aspect of the present disclosure to provide an ultrasound imaging apparatus capable of displaying a diagnostic image together with an image indicator indicating a target part by synchronizing the diagnostic image of the ultrasound imaging apparatus with a reference imaging, and a method of controlling the same.

Additional aspects of the present disclosure will be set forth in part in the description which follows and, in part, will be obvious from the description, or may be learned by practice of the invention.

In accordance with one aspect of the present disclosure, an ultrasound imaging apparatus includes a receiving unit receiving an imaging signal of an object, a display unit displaying a diagnostic image on a first section based on the imaging signal and displaying an image indicator indicating a diagnostic area of the object on a second section, and a controlling unit synchronizing the diagnostic image with a reference image corresponding to the diagnostic image, wherein the image indicator comprises a target organ marker which comprises a three dimensional volume image corresponding to the object and a three dimensional beam direction marker on the target organ, and the three dimensional beam direction marker indicates a direction of an ultrasound probe beam.

The display unit may display a plurality of reference images similar to the diagnostic image on a third section, the ultrasound imaging apparatus may further include an input unit receiving a selection of one reference image among the plurality of reference images from users, and the controlling unit may synchronize the diagnostic image with the selected reference image.

The image indicator may further include a body axis marker indicating an anatomical location of the object.

The ultrasound imaging apparatus may further include an input unit receiving user requests including a selection of a target part and a diagnostic location from users, and the display unit may further display the target organ marker the second section according to the selection of the target part and the diagnostic location.

The reference image may include a cross-sectional image according to a diagnostic mode of the three dimensional volume image.

The ultrasound imaging apparatus may further include a storage unit storing the reference images according to target parts and diagnostic locations of an object, and an input unit receiving a selection of the target part and the diagnostic location from the user.

The controlling unit may set the image indicator as being in a reference position and a reference location when the diagnostic image is synchronized with the reference image.

The receiving unit may receive signals of a relative position of the ultrasound probe relative to the reference position and a relative location of the ultrasound probe relative to the reference location, and the controlling unit may move the image indicator according to the relative position and the relative location.

The receiving unit may receive a first image signal and a second image signal of the object, the display unit may display a first diagnostic image based on the first image signal and a second diagnostic image based on the second image signal on the first section, and the controlling unit may move the image indicator according to a relative position and a relative location of the second diagnostic image relative to a position and a location of the first diagnostic image.

The display unit may display the diagnostic area on the second section by moving the target organ marker.

The ultrasound imaging apparatus may further include an ultrasound probe transmitting an ultrasound to the object, receiving an echo ultrasound reflected from the object, and transforming the echo ultrasound to an electrical signal, and the display unit may display the diagnostic image of the object on a first section based on the electrical signal.

The ultrasound probe may include a detecting unit, wherein the detecting unit may detect a relative position of the ultrasound probe relative to a reference position and a relative location of the ultrasound probe relative to a reference location, and the controlling unit may move the image indicator according to the relative position and the relative location.

The detecting unit may include at least one of an angular velocity sensor, a magnetic sensor, an acceleration sensor, a gravity sensor, and a gyro sensor.

The ultrasound imaging apparatus may further include a storage unit storing at least one reference image.

The ultrasound imaging apparatus may further include an input unit receiving a change signal for changing the reference image from users, and the controlling unit may synchronize the changed reference image based on the change signal.

In accordance with another aspect of the present disclosure, a controlling method of an ultrasound imaging apparatus includes receiving an image signal of an object, displaying a diagnostic image on a first section based on the image signal, synchronizing the diagnostic image with a reference image corresponding to the diagnostic image, and displaying an image indicator indicating a target part of the object on a second section, wherein the image indicator comprises a target organ marker which comprises a three dimensional volume image corresponding to the object and a three dimensional beam direction marker on the target organ, and the three dimensional beam direction marker indicates a direction of an ultrasound probe beam..

The method may further include displaying at least one reference images similar to the diagnostic image on a third section before the synchronizing, and receiving a selection of one reference image among the at least one reference images from the user, wherein the synchronizing is that the selected reference image by the user is synchronized with the diagnostic image.

The reference image may include a cross-sectional image according to a diagnostic mode of the three dimensional volume image.

### BRIEF DESCRIPTION OF THE DRAWINGS

These and/or other aspects of the disclosure will become apparent and more readily appreciated from the following description of embodiments, taken in conjunction with the accompanying drawings of which:
FIG. 1 is a perspective view illustrating an ultrasound imaging apparatus according to an embodiment of the present disclosure;
FIG. 2 is a control block diagram illustrating the ultrasound imaging apparatus according to an embodiment of the present disclosure;
FIGS. 3 and 4 are views illustrating a control operation by a controlling unit;
FIGS. 5 (a) and (b) are views illustrating a reference image stored in a storage unit according to an embodiment of the present disclosure;
FIGS. 6 and 7(a) and (b) are views illustrating an image indicator displayed on a second section of a display unit;
FIGS. 8 (a) to (d) and 9 (a) to (d) are views illustrating a diagnostic image and an image according to changing a location and a position of an ultrasound probe; and
FIG. 10 is a flow chart illustrating a control method of an ultrasound imaging apparatus according to another embodiment of the present disclosure.

### DETAILED DESCRIPTION

The present disclosure will now be described more fully with reference to the accompanying drawings, in which exemplary embodiments of the disclosure are shown. The disclosure may, however, be embodied in many different forms and should not be construed as being limited to the embodiments set forth herein; rather, these embodiments are provided so that this disclosure will be thorough and complete, and will fully convey the concept of the disclosure to those skilled in the art. Like reference numerals in the drawings denote like elements, and thus their description will be omitted. In the description of the present disclosure, if it is determined that a detailed description of commonly-used technologies or structures related to the embodiments of the present disclosure may unnecessarily obscure the subject matter of the invention, the detailed description will be omitted. It will be understood that, although the terms first, second, third, etc., may be used herein to describe various elements, components, regions, layers and/or sections, these elements, components, regions, layers and/or sections should not be limited by these terms. These terms are only used to distinguish one element, component, region, layer or section from another region, layer or section.

Embodiments of the present disclosure will now be described with reference to accompanying drawings.

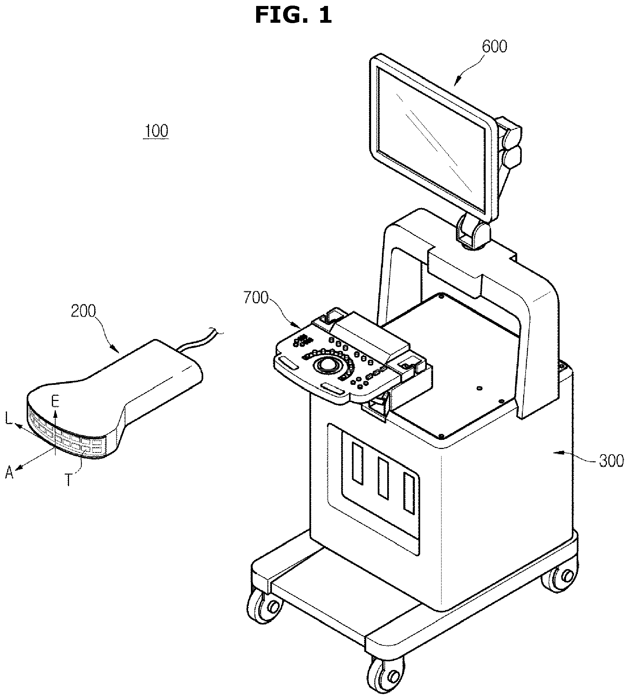

FIG. 1 is a perspective view illustrating an ultrasound imaging apparatus according to an embodiment of the present disclosure.

As illustrated in FIG. 1, an ultrasound imaging apparatus 100 may include an ultrasound probe 200, a main body 300, a display unit 600 and an input unit 700.

The ultrasound probe 200 is connected to an end of a cable and the other end of the cable may be connected to a male connector (not shown). The male connector may be physically coupled with a female connector (not shown) of the main unit 300.

The ultrasound probe 200 may include at least one transducer T, and use them to transmit ultrasound signals to an object and receive echo ultrasounds reflected from the object. The at least one transducer T may form at least one row at one end of the ultrasound probe 200, as shown in FIG. 1.

The object may be a living body of a human or animal, an organ in the living body, such as blood vessels, bones, muscles, etc., but is not limited thereto. Therefore, anything whose internal structure may be imaged by the ultrasound imaging apparatus 100 may be the object.

Three directions perpendicular to one and another based on a center of the transducer may be defined as an axis direction (A), a lateral direction (L), and an elevation direction (E). Particularly, a direction in which an ultrasound is radiated may be defined as the axis direction (A), a direction in which transducers are formed in a row may be defined as the lateral direction (L), and a direction perpendicular to the axis direction and the lateral direction may be defined as the elevation direction (E).

The main body 300 may include a main component of the ultrasound imaging apparatus 100, such as a transmit signal generator (not shown). When an examiner inputs an ultrasound diagnosis command, the transmit signal generator (not shown) may generate a transmit signal and transmit the transmit signal to the ultrasound probe 200.

The main body 300 may include at least one female connectors (not shown), which are physically coupling to male connectors (not shown) connected to cables so that the main body 300 and the ultrasound probe 200 may communicate signals. For example, a transmit signal generated by the transmit signal generator may be sent to the ultrasound probe 200 through the male connector connected to the female connector of the main body 300 and the cable.

In addition, a plurality of casters configured to fix or move the ultrasound imaging apparatus 100 to a certain place may be mounted on a lower portion of the main body 300.

The input unit 700 may receive commands related to an operation of the ultrasound imaging apparatus 100 from users. For example, the user may input a command to start ultrasonic examination, select a target part, select a diagnostic position, select a mode for an output ultrasonic image, select a reference imaging, etc., through the input unit 700. The command may be performed by selecting one of user interfaces displayed on the display unit 600.

The input unit 700 may receive changing geometry information from the user, wherein the changing geometry information may be changing zoom, inversing, reversing, rotating, or changing focus of an ultrasound image. To match a reference image with a diagnostic image, the reference image displayed on a third section of the display unit 600, which is described later, may be changed zoom thereof, inversed, reversed, rotated or changed focus thereof according to the changing geometry information input by the user.

The target part may be referred to as a part of an object related to image signals currently receiving, and may include all organs, e.g., a heart, a brain, a breast, which are examined by the ultrasound imaging apparatus. By inputting a part to be examined in advance, the user may easily obtain information related to a diagnostic image such as a reference image corresponding to the diagnostic image.

The diagnostic position may be information about the target part of the object is examined from where, which is input by the user through the input unit 700. For example, through the input unit 700, the user may input whether a heart is examined from an esophagus or from ribs. Particularly, when examining a brain, a diagnostic position may be Mid-Sagittal plane, Trans-ventricular plane, Trans-thalamic plane, and Trans-cerebellar plane, and when examining a heart, a diagnostic position may be Four-chamber view, Five chamber view, three vessel view (3VT), Right ventricular outflow tract (RVOT), Left ventricular outflow tract, (LVOT), Bicaval View, Aortic Arch, Ductal Arch, Short Axis View, and Long Axis view.

Modes for an ultrasound imaging may include an Amplitude mode (A-mode), a Brightness mode (B-mode), a Doppler mode (D-mode), an Elastography mode (E-mode), a Motion mode (M-mode), etc,.

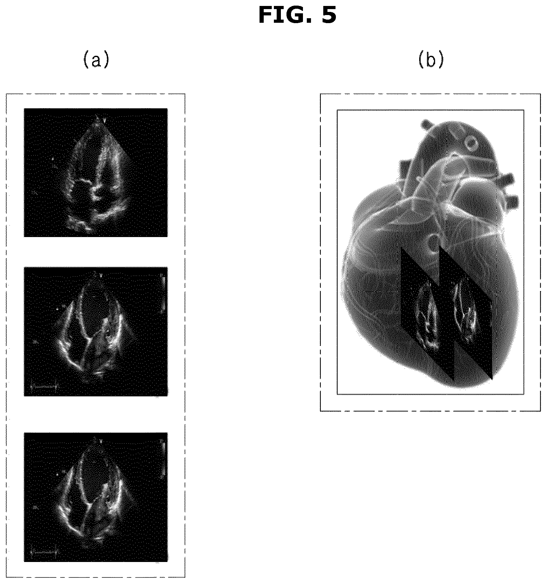

A reference image may be a reference for a target part of an object and a diagnostic position, and the user may guess where are the target part and the diagnostic position on the basis of the reference image. The reference image may be a cross-sectional view of a three dimensional volume image of the target part, which is described later with reference with FIG. 5. At least one reference images may be provided to the user through the display unit 600, and the user may select one reference image through the input unit 700. The selected reference image may be synchronized with the diagnostic image.

Commands input through the input unit 700 may be transmitted to the main body 300 through wired/wireless communication.

The 'user' as used herein may be a medical person who performs diagnosis with the ultrasound imaging apparatus 100, including a doctor, a radiographer, a nurse, etc., but is not limited thereto and thus may be anyone who uses the ultrasound imaging apparatus 100.

The input unit 700 may include at least one of a keyboard, mouse, trackball, touch screen, foot switch, and foot pedal, but is not limited thereto.

The input unit 700 may be provided on an upper portion of the main body 300 as shown in FIG. 1, or may be provided on a lower portion of the main body 300 when the input unit 700 is implemented with a foot switch or a foot pedal.

If the input unit 700 is implemented in a Graphical User Interface (GUI), i.e., in software like a touch screen, the input unit 700 may be displayed on the display unit 600, which will be described later.

At least one ultrasound probe holders may be provided on around the input unit 700 to hold the ultrasound probe 200. Therefore, the user may keep the ultrasound probe 200 in the ultrasound probe holder while the ultrasound imaging apparatus 100 is not used.

The display unit 600 displays an image obtained in an ultrasonic diagnostic process. The display unit 600 displays the image according to a mode selected by the user. If no mode is selected, the image may be displayed in a default mode set by the user in advance, e.g., B mode.

Particularly, the display unit 600 may display a diagnostic image on a first section, and display an image indicator showing a target part of the object on a second section. The display unit 600 may display at least one reference images similar to the diagnostic image on a third section.

The first section may be a section for displaying the diagnostic image of the object being diagnosed currently, and other images may be displayed according to ultrasound imaging modes, the type of ultrasound probe. The diagnostic image may include a three-dimensional (3D) image.

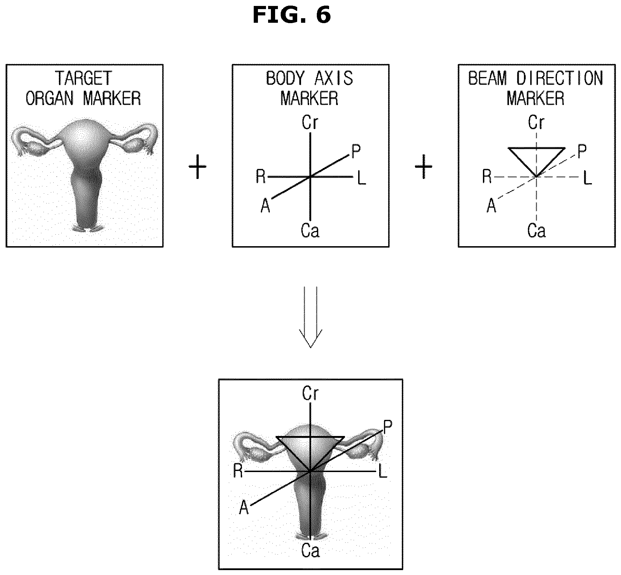

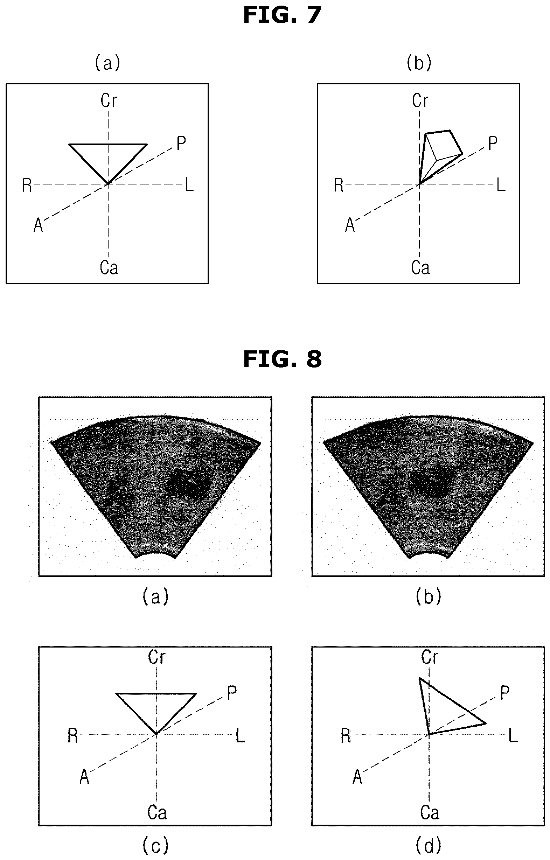

The second section may be a section for displaying an image indicator for indicating the target part. For example, an organ imaging virtually formed and a beam direction marker showing a direction of an ultrasound probe beam may be displayed. A target part may be directly displayed to the user, which is will be described later with reference with FIGS. 6 and 7.

The third section may be a section for displaying at least one reference imaging, which is determined to be similar to the diagnostic image. The user may select a single reference imaging among the displayed reference imaging through the input unit 700.

The user may input a change signal for a reference imaging through the input unit 700. The change signal for a reference imaging may allow a controlling unit 400 to change a reference imaging determined to be most similar to the diagnostic image or a reference imaging selected by the user to another reference imaging. Therefore, the controlling unit 400 may synchronize a changed reference imaging according to the signal for changing. The change signal may serve as a revise signal and input by the user even after the synchronization. The changed reference imaging may be the second most similar reference imaging next to the most similar reference imaging, or a reference imaging selected by the user.

The display unit 600 may be mounted by coupling to the main body 300. However, the display unit 600 may be separated from the main body 300. Although not illustrated in FIG. 1, a sub display for displaying an application (e.g., menus and guidance needed for ultrasonic diagnosis) related to operations of the ultrasound imaging apparatus 100 may be provided.

The display unit 600 may employ a Liquid Crystal Display (LCD), a Light Emitting Diodes (LED), an Organic Light Emitting Diodes (OLED), an Active Matrix Organic Light Emitting Diodes (AMOLED), a flexible display, a three dimensional 3D display (3D) and a touch screen having a display function and an input function.

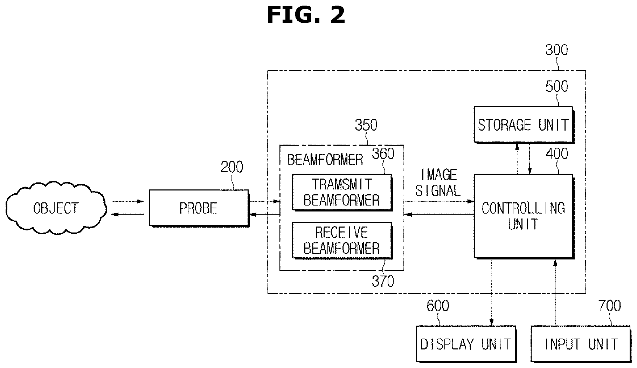

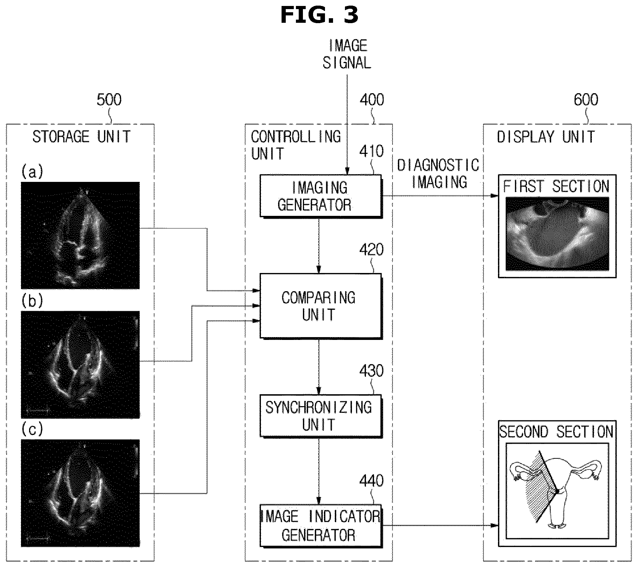

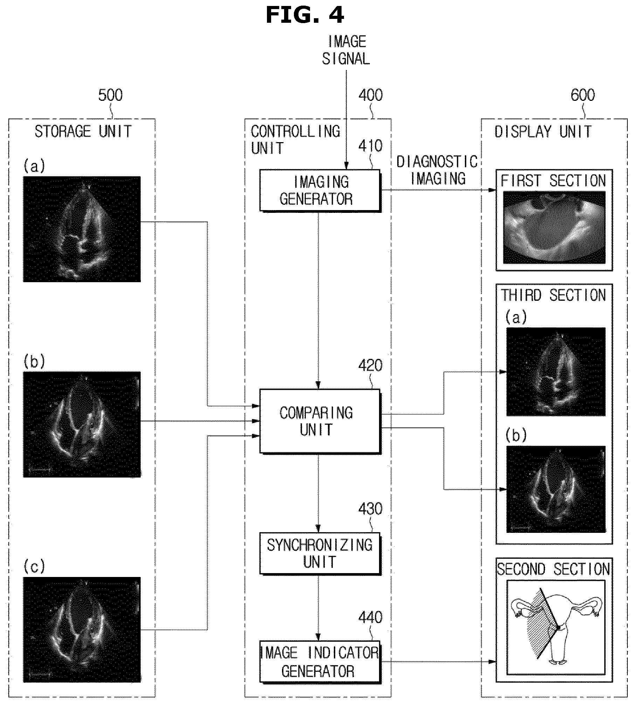

FIG. 2 is a control block diagram illustrating the ultrasound imaging apparatus according to an embodiment of the present disclosure, and FIGS. 3 and 4 are views illustrating a control operation by a controlling unit.

Referring to FIG. 2, the ultrasound imaging apparatus 100 may image an inside or an outside of an object by the ultrasound probe 200, a beamformer 350, a controlling unit 400, a storage unit 500, the input unit 700, and the display unit 600.

The ultrasound probe 200 may include at least one transducer T to transmit ultrasounds to an object, receive echo ultrasounds reflected from the object, and perform transformation between electric signals and ultrasounds.

Particularly, when the ultrasound probe 200 is supplied with power from an external power supply or an internal electricity storage device, e.g., battery, the transducers generate ultrasounds while vibrating due to the applied current and irradiate the ultrasounds to an external object. Each transducer receives an echo ultrasound reflected and returned from the object, and generates a current while vibrating due to the echo ultrasound, the current having a frequency corresponding to the vibration frequency.

The transducer T may be a Magnetostrictive Ultrasound Transducer (MUT) that uses magnetostrictive effects of a magnetic substance, a Capacitive Micromachined Ultrasonic Transducer (cMUT) that uses vibration of hundreds or thousands of microfabricated thin films, or a Piezoelectric Ultrasonic Transducer (PUT) that uses piezoelectric effects of a piezoelectric substance.

The transducers T may be linear array, convex array, phased array, sector array transducers, etc., which may be arranged in a form of a row or a matrix. When the transducers T are arranged in a row, they may be swung in the elevation direction to obtain multiple ultrasonic images; and when they are arranged in a form of a matrix, multiple ultrasonic images may be obtained only with a single transmission of ultrasounds.

However, the transducers are not limited thereto, but may be implemented with any other types of transducers known to skilled people in the art.

Although not shown, the ultrasound probe 200 may further include a detecting unit (not shown), and the detecting unit may be disposed in the case of the ultrasound probe 200. The detecting unit may generate information about position and location of the ultrasound probe 200 by detecting a position and a location of the ultrasound probe 200. The detecting unit may employ an angular velocity sensor, a magnetic sensor, an acceleration sensor, a gravity sensor, and a gyro sensor, but is not limited thereto. That is, the detecting unit may employ any kind of sensor capable of detecting a second or a three dimensional position and location of the ultrasound probe 200.

The beamformer 350 may include a transmit beamformer 360 and a receive beamformer 370 to perform transformation between analog and digital signals and to adjust time differences of ultrasounds transmitted by or received from the at least one transducers T.

Ultrasounds adjusted time differences may be focused as a receive signal (S), and the focused receive signal (S) may be supplied to the controlling unit 400. As mentioned above, the signal supplied to the controlling unit 400 may be defined as an imaging signal.

The storage unit 500 may store reference images according to target parts and diagnostic location. Particularly, the storage unit 500 may store various reference images according to diagnostic locations based on one target part or may store various reference images according to target parts. In addition, the storage unit 500 may store image indicators set up according to each reference images.

FIG. 5 is a view illustrating a reference image stored in a storage unit according to an embodiment of the present disclosure.

A reference image may be a reference for a target part of an object and a diagnostic position, and the user may guess where are the target part and the diagnostic position on the basis of the reference image. When referring to FIG. 5a, the reference image stored in the storage unit 500 may be a cross-sectional view of the target part and the diagnostic position, and when referring to FIG. 5b, the reference image stored in the storage unit 500 may be a cross-sectional view of a three dimensional volume image of the target part and the diagnostic position, wherein the three dimensional volume image may correspond to the target part and the cross-sectional view may correspond to the diagnostic location. That is, the storage unit 500 may store a cross-sectional view, such as FIG. 5a, a three dimensional volume image and a cross-sectional view of a three dimensional volume image, such as FIG. 5b.

FIGS. 6 and 7 are views illustrating an image indicator displayed on a second section of a display unit.

As illustrated in FIG. 6, the image indicator may include a target organ marker, a body axis marker, and an ultrasound beam direction marker. The display unit 600 may virtually display a target part by these markers.

The target organ marker may display a target part of an object, such as a chest, a liver, an abdomen, an uterus, an anus, etc., and may be identical to a three dimensional volume image of a reference image. The body axis marker may display an anatomical location, such as Cranial (Cr), Caudal (Ca), Anterior (A), Posterior (P), Right (R) and Left (L), by using a three dimensional coordinate system. Hereinafter, the body axis marker may be displayed by using a three dimensional rectangular coordinate system, but is not limited thereto. The body axis marker may be displayed in various shapes. The ultrasound beam direction marker may display a direction of an ultrasound beam. The ultrasound beam direction marker may have a square shape in a leaner ultrasound probe, a fan shape having trapezoid shaped in a convex ultrasound probe or a vaginal ultrasound probe, a fan shape in a phased array ultrasound probe, and a three-dimensional shape of a cross-sectional beam of each ultrasound probe in a two-dimensional matrix ultrasound probe (2D Matrix ultrasound probe), and may display the transmission direction of the ultrasound beam. According to one embodiment, the ultrasound beam direction marker may be displayed with the body axis marker so that a vertex of a triangle in the ultrasound beam direction marker is to be located at an origin of a coordinate system of the body axis marker. In addition, when the ultrasound probe 200 is a one dimensional array ultrasound probe (1D), the ultrasound beam direction marker may be displayed in two dimensions, and when the ultrasound probe 200 is a two dimensional array ultrasound probe or a three dimensional mechanical ultrasound probe, the ultrasound beam direction marker may be displayed in three dimensional volume.

The storage unit 500 may store reference images according to a target part and a diagnostic position in advance. A diagnostic image and the stored reference image may be subject to be compared by a comparing unit 430 of the controlling unit, which is described later.

The storage unit 500 may store the image indicator in association with the target part and the diagnostic location of the object. For example, the storage unit 500 may store the image indicator including a target organ marker, a body axis marker and an ultrasound beam direction marker, wherein the image indicator correspond to the target part and the diagnostic location of the object, as illustrated in FIG. 6. When the target organ marker and the ultrasound beam direction marker may be displayed in a two dimensional when using a two dimensional coordinate system, as illustrated in FIG. 7a. When the target organ marker and the body axis marker may be displayed in three dimensions when using a three dimensional coordinate system, as illustrated in FIG. 7b. The storage unit 500 may store reference images in association with image indicators.

The storage unit 500 may include a program part and a data part.

The program part may include Operating system (OS) booting programs to control overall operations of the ultrasound imaging apparatus 100. The program part may include programs related to the operation of the controlling unit 400. The data part may be a place where data generated according to the operation of the ultrasound imaging apparatus 100, such as the reference image and the image indicator, is stored.

The storage unit 500 may employ volatile memory, such as, cache memory, Read Only Memory (ROM), Programmable ROM (PROM), Erasable Programmable ROM (EPROM), Electrically Erasable Programmable ROM (EEPROM) and Flash memory, nonvolatile memory, such as, Random Access Memory (RAM), Hard Disk Drive (HDD), or CD-ROM, but is not limited thereto.

The controlling unit 400 may control overall operations of the ultrasound imaging apparatus 100. Particularly, the controlling unit 400 may generate control signals to control at least one of the transmit beamformer 360, the receive beamformer 370, the storage unit 500, and the display unit 600 to response commands input through the input unit 700. The controlling unit 400 may generate control signals to control compartments to response commands received from outside devices through wired/wireless communication.

Referring to FIGS. 3 and 4, the controlling unit 400 may include an imaging generator 410, a comparing unit 420, a synchronizing unit 430, and an image indicator generator 440.

The imaging generator 410 may generate a diagnostic image based on an electrical signal, hereinafter, an imaging signal, received from the beamformer 350, and may allow the display unit 600 to display the diagnostic image on the first section of the display unit 600. The generated diagnostic image may be an Amplitude mode (A-mode), a Brightness mode (B-mode), a Doppler mode (D-mode), an Elastography mode (E-mode), and a Motion mode (M-mode), but is not limited thereto. Hereinafter, an image in Brightness mode (B-mode) will be described as an example. The B-mode may be a diagnosis mode in which size of echo ultrasounds reflected from the object is converted to brightness and displayed. According to embodiments, diagnostic images may be displayed in various modes. In addition, the diagnostic image may be generated in two or three dimensional image. Particularly, the image generator 410 may generate diagnostic image based on focused ultrasound signal through the receive beamformer 370.

The comparing unit 420 may compare the generated diagnostic image with at least one reference image stored in the storage unit 500. Referring to FIG. 3, the comparing unit 420 may select a reference image which is the most similar with a diagnostic image after comparing a diagnostic image with at least one reference image (a, b, c) corresponding to a target part and a diagnostic location, both of which are input by the user. In addition, the comparing unit 420 may automatically select the most similar reference image, as illustrated in FIG. 3. However, the comparing unit 420 may allow the user to select the most similar image after displaying at least one reference image (a, c) determined to be the most similar image among the reference image (a, b, c) corresponding to the target part and the diagnostic location, as illustrated in FIG. 4.

The synchronizing unit 430 may synchronize the selected reference image with the diagnostic image, and may set a diagnostic area of the image indicator as a reference position and a reference location. The synchronizing may be defined as associating the selected reference image with the diagnostic image. Particularly, the synchronizing unit 430 may synchronize the selected reference image with the diagnostic image, and may set a position and a location of the ultrasound probe 200 at synchronizing point as a reference position and reference location of the ultrasound probe 200. The set reference location may be a location to allow the center of gravity of the ultrasound probe 200 to be an original point of a coordinate system of the body axis marker, and the set reference position may be an angle according to an orientation of the ultrasound probe 200 detected by the detecting unit. The reference position and the reference location may be stored in the storage unit 500.

The image indicator generator 440 may generate an image indicator displayed on the second section based on the reference position and the reference location.

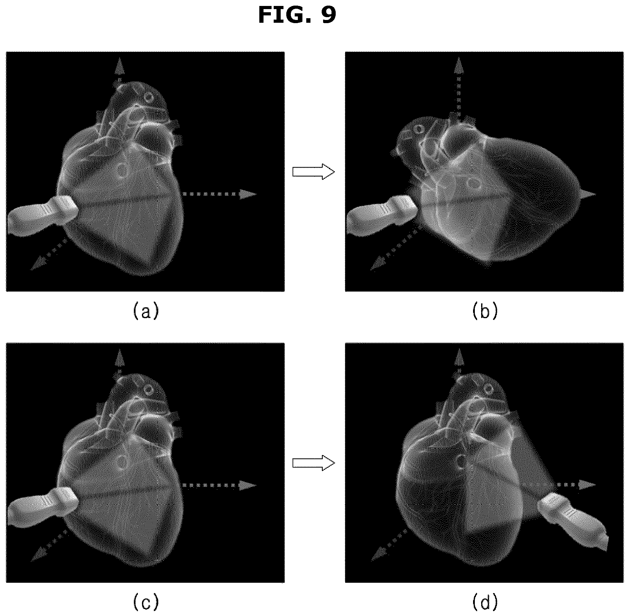

FIGS. 8 and 9 are views illustrating a diagnostic image and an image according to changing a location and a position of an ultrasound probe.

Particularly, referring to FIG. 8, when a diagnostic image, as illustrated in (a), may be set as a reference position and a reference location, the image indicator generator 440 may generate an image indicator, as illustrated in (c). When a position and location of the ultrasound probe 200 is changed, the diagnostic image may be displayed in the first section, as illustrated in (b) and the image indicator generator 440 may generate an image indicator, as illustrated in (d), according to a relative position and a relative location of the ultrasound probe 200 based on the reference position and the reference location. That is, an image indicator may be moved according to a relative position and a relative location based on the reference position and the reference location of the ultrasound probe 200.

The relative position and the relative location based on the reference position and the reference location of the ultrasound probe 200 may be detected by a sensor of the detecting unit of the ultrasound probe 200, and a relative position and a relative location estimated from a diagnostic image, as illustrated in FIG. 9.

The image indicator generator 440 may generate an image indicator in a way that the beam direction marker is displayed in the body marker coordinate system, as illustrated in FIG. 8, in a way that a direction of the target organ marker may be changed, as illustrated in (a, b) of FIG. 9, or in a way that a point of the beam direction marker is changed, as illustrated in (c, d) of FIG. 9, but is not limited thereto. The image indicator may be displayed in various ways capable of showing a position and location of an ultrasound probe.

The controlling unit 400 may include a processor, a ROM in which control programs are stored, and a RAM in which signals or data input from the outside are stored or which is used as a storage area to correspond to various operations

The controlling unit 400 may control overall operations of the ultrasound imaging apparatus 100 and a flow of signal between compartments disposed inside the ultrasound imaging apparatus 100, and may process data. The controlling unit 400 may control power supplied to the internal compartments.

When conditions input by the user or stored in advance are met, the controlling unit 400 may perform Operation System (OS) and various applications. Operations of the controlling unit 400 may be performed by being programed by the storage unit 500. The description about the storage unit 500 is mentioned above, so it is omitted.

The processor may be realized in a shape of System on Chip (SoC) including core and GPU. The processor may include a single core, a dual core, a triple core, a quad core and various multiple cores. The controlling unit 400 may include a graphic processing board which is a circuit board electrically connected includes a processor, and RAM or ROM. The process, a ROM, and RAM may be connected to each other through an internal bus. The controlling unit 400 may be referred as a compartment including a processor, a ROM, and a RAM. The controlling unit 400 may be referred as a compartment including a processor, a ROM, a RAM, and a processing board.

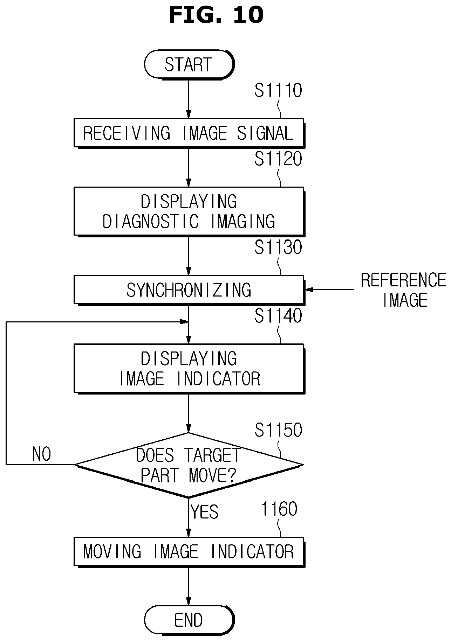

FIG. 10 is a flow chart illustrating a control method of an ultrasound imaging apparatus according to another embodiment of the present disclosure.

The ultrasound imaging apparatus 100 may irradiate ultrasounds to the object, detect reflected signals from the object, and then obtain imaging signals (S 1110).

The reflected signals may be ultrasound signals received from the transducer, and may be changed to electronic signals by the beamformer. The electrical signals may be input to the controlling unit as imaging signals and then may be displayed on the first section of the display unit as a diagnostic image (S1120).

The diagnostic image and a reference image may be synchronized (S1130), and during the synchronization, the diagnostic image may be compared with at least one reference images stored in the storage unit, and then the most similar reference image to the diagnostic image, which is identical to the diagnostic image, may be selected. In this case, the selection of the reference image may be performed by estimating by ultrasound imaging apparatuses, or by selecting a single reference image through the input unit by displaying at least one reference images on the display unit.

A reference image may be a reference for a target part of an object and a diagnostic position, and the user may guess where are the target part and the diagnostic position on the basis of the reference image. The reference image may be a cross-sectional view of a three dimensional volume image of the target part or may be stored to correspond to the target part and the diagnostic location of the object.

The synchronization may represent associating the selected reference image with the diagnostic image.

After the synchronization of the selected reference image and the diagnostic image, a position and location of the ultrasound probe at a time of the synchronization may be set as the reference position and location of the ultrasound probe. The set reference position may allow the center of gravity of the ultrasound probe to be an origin of the body axis marker coordinate system, the reference position may be an angle according an orientation of the ultrasound probe detected by the detecting unit, and then the image indicator may be displayed on the second section of the display unit (S 1140). At this time, the ultrasound beam direction marker may be displayed together with the body axis marker to allow a start point of an ultrasound to be placed on the origin of the body axis marker coordinate system. The beam direction marker and the body axis marker, which is information to virtually display the diagnostic area in the second section, may be displayed together with the target organ marker displaying the target part of the objet in two dimensions or three dimensions.

When the target part of the object is changed according to the movement of the ultrasound probe (S 1150), the diagnostic image displayed on the first section may be changed. Accordingly, the image indicator may be moved (S1160). In this case, the position and the location of the ultrasound probe may be determined based on a detected signal by the detecting unit provided in the ultrasound probe and may be determined based on the changed diagnostic image of the object. Particularly, when the position and the location of the ultrasound probe are detected by an angular velocity sensor, a magnetic sensor, an acceleration sensor, a gravity sensor, and a gyro sensor, all of which are provided in the ultrasound probe, the image indicator may be changed from the reference position and the relative location to a relative position and a relative location. In addition, as mentioned above, the reference image, which is the most similar to the changed diagnostic image, may be selected, and the ultrasound imaging apparatus may estimate the relative position and the relative location based on the relation between reference information about the reference image of the changed diagnostic image and a preset reference position and location, and accordingly the image indicator may be moved. According to one exemplary embodiment of the movement of the image indicator, a beam direction of the beam direction marker may be changed.

As is apparent from the above description, the ultrasound imaging apparatus and a control method thereof may provide an accurate position and direction of the ultrasound probe diagnosing the object to the user by using the image indicator.

Without requiring the user's judgment, a target part virtually displayed is synchronized with a reference image so that the convenience may be provided to the user.

By matching the target organ marker configured to display the target part of the object, the body axis marker configured to display the diagnostic position of the object, and the beam direction marker configure to display a direction of an ultrasound probe beam received from the ultrasound probe with the target part currently examined by the user, the user may intuitively and accurately recognize the position of the ultrasound probe.

According to embodiments of the present disclosure, some components provided in the ultrasound imaging apparatus 100 may be implemented as modules. Here, the term "module" may represent a software element or a hardware element, such as a Field Programmable Gate Array (FPGA) or an Application Specific Integrated Circuit (ASIC), and the module may perform a predetermined role. However, the module is not limited to software or hardware. Further, the module may be constructed to exist in an addressable storage module, or to play one or more processors.

The module may include elements (e.g., software elements, object-oriented software elements, class elements and task elements), processors, functions, properties, procedures, subroutines, segments of a program code, drivers, firmware, a microcode, a circuit, data, a database, data structures, tables, arrays, and variables. Herein, functions provided by components and modules may be provided by a smaller number of combined larger components and modules, or by a larger number of divided smaller components and modules. In addition, the components and modules may be realized to operate one or more CPUs in a device.

The ultrasound imaging apparatus 100 and the control method thereof may be implemented as a computer code on a computer readable recording medium. The computer readable recording medium may include various kinds of recording medium stored data decrypted by the computer system. For example, there may be a Read Only Memory (ROM), a Random Access Memory (RAM), a magnetic tape, a magnetic disk, a flash memory, and an optical data storage device. In addition, the medium may be distributed to computer systems over a network, in which computer-readable code may be stored and executed in a distributed manner.

Although a few embodiments of the present disclosure have been shown and described, it would be appreciated by those skilled in the art that changes may be made in these embodiments without departing from the principles and spirit of the disclosure, the scope of which is defined in the claims and their equivalents.

## Claims

1. An ultrasound imaging apparatus (100) comprising:
an ultrasound probe (200) configured to generate a three-dimensional imaging signal of an object by transmitting an ultrasound beam, wherein the ultrasound probe (200) comprises a detecting unit configured to detect a relative position of the ultrasound probe (200) relative to a reference position and a relative location of the ultrasound probe (200) relative to a reference location;
a display unit (600) configured to display a diagnostic image in a first section based on the imaging signal, and display an image indicator indicating a target part of the object in a second section; and
a controlling unit (400) configured to synchronize the diagnostic image with a selected reference image corresponding to the diagnostic image and to set the position and location of the ultrasound probe at the time of synchronization as the reference position and location of the ultrasound probe,
wherein the reference image comprises a cross-sectional image of a three dimensional volume image;
wherein the image indicator comprises a target organ marker which is configured to display the target part as a three dimensional volume image identical to the three dimensional volume image of the reference image and to display a beam direction marker which indicates the direction of the ultrasound probe beam in three dimension; and
wherein the display unit is configured to display a plurality of reference images similar to the diagnostic image in a third section, the ultrasound imaging apparatus further comprises an input unit (700) configured to receive a selection of one image as the selected reference image among the plurality of reference images from a user, and the controlling unit (400) is configured to move the image indicator according to the relative position and the relative location.

2. The ultrasound imaging apparatus (100) of claim 1, wherein
the image indicator further comprises a body axis marker indicating an anatomical location of the object.

3. The ultrasound imaging apparatus (100) of claim 1, the input unit (700) is further configured to receive a selection of the target part and a diagnostic location from a user, wherein the display unit (600) is further configured to display the target organ marker in the second section according to the selection of the target part and the diagnostic location.

4. The ultrasound imaging apparatus (100) of claim 1, further comprising:
a storage unit (500) configured to store the reference images of the target parts
and diagnostic locations of an object, and the input unit (700) is configured to receive a selection of the target part and a diagnostic location from the user.

5. The ultrasound imaging apparatus (100) of claim 1, wherein the detecting unit comprises at least one of an angular velocity sensor, a magnetic sensor, an acceleration sensor, a gravity sensor, and a gyro sensor.

6. A controlling method of the ultrasound imaging apparatus (100) according to claim 1 comprising:
generating three-dimensional imaging signal of an object;
displaying a diagnostic image in the first section based on the imaging signal;
displaying a plurality of reference images similar to the diagnostic image in the third
section before the synchronizing;
receiving a selection of one image as the selected reference image among the plurality of reference images from a user;
synchronizing the diagnostic image with the selected reference image corresponding to the diagnostic image and setting the position and location of the ultrasound probe at the time of synchronization as the reference position and location of the ultrasound probe; and
displaying the image indicator indicating the target part of the object and the direction of the ultrasound probe beam in the second section,
detecting a relative position of the ultrasound probe (200) relative to the reference position and a relative location of the ultrasound probe (200) relative to the reference location; and
moving the image indicator according to the relative position and the relative location.

## Patentansprüche

1. Ultraschallbildgebungsvorrichtung (100), welche Folgendes aufweist:
eine Ultraschallsonde (200), die dafür vorgesehen ist, ein dreidimensionales Bildgebungssignal eines Objekts durch Übertragen eines Ultraschallstrahls zu erzeugen, wobei die Ultraschallsonde (200) eine Detektiereinheit aufweist, die dafür vorgesehen ist, eine relative Position der Ultraschallsonde (200) relativ zu einer Referenzposition und einen relativen Standort der Ultraschallsonde (200) relativ zu einem Referenzstandort zu detektieren;
eine Anzeigeeinheit (600), die dafür vorgesehen ist, ein Diagnosebild in einem ersten Abschnitt basierend auf dem Bildgebungssignal anzuzeigen, und einen Bildindikator, der ein Zielteil des Objekts in einem zweiten Abschnitt kennzeichnet, anzuzeigen; und
eine Steuereinheit (400), die dafür vorgesehen ist, das Diagnosebild mit einem ausgewählten Referenzbild, das mit dem Diagnosebild korrespondiert, zu synchronisieren, und die Position und den Standort der Ultraschallsonde zu der Zeit der Synchronisierung als die Referenzposition und den Referenzstandort der Ultraschallsonde festzulegen,
wobei das Referenzbild ein Querschnittsbild eines dreidimensionalen Volumenbilds aufweist;
wobei der Bildindikator eine Zielorganmarkierung aufweist, die dafür vorgesehen ist, den Zielteil als ein dreidimensionales Volumenbild identisch zu dem dreidimensionalen Volumenbild des Referenzbilds anzuzeigen, und eine Strahlrichtungsmarkierung anzuzeigen, welche die Richtung des Ultraschallsondenstrahls dreidimensional kennzeichnet; und
wobei die Anzeigeeinheit dafür vorgesehen ist, eine Vielzahl von Referenzbildern ähnlich zu dem Diagnosebild in einem dritten Abschnitt anzuzeigen, wobei die Ultraschallbildgebungsvorrichtung des Weiteren eine Eingabeeinheit (700) aufweist, die dafür vorgesehen ist, eine Auswahl eines Bilds als das ausgewählte Referenzbild unter der Vielzahl von Referenzbildern von einem Benutzer zu empfangen, und wobei die Steuereinheit (400) dafür vorgesehen ist, den Bildindikator gemäß der relativen Position und dem relativen Standort zu bewegen.

2. Ultraschallbildgebungsvorrichtung (100) nach Anspruch 1, wobei der Bildindikator des Weiteren eine Körperachsenmarkierung aufweist, die einen anatomischen Standort des Objekts kennzeichnet.

3. Ultraschallbildgebungsvorrichtung (100) nach Anspruch 1, wobei die Eingabeeinheit (700) des Weiteren dafür vorgesehen ist, eine Auswahl des Zielteils und einen Diagnosestandort von einem Benutzer zu empfangen, wobei die Anzeigeeinheit (600) des Weiteren dafür vorgesehen ist, die Zielorganmarkierung in dem zweiten Abschnitt gemäß der Auswahl des Zielteils und des Diagnosestandorts anzuzeigen.

4. Ultraschallbildgebungsvorrichtung (100) nach Anspruch 1, welche des Weiteren Folgendes aufweist:
eine Speichereinheit (500), die dafür vorgesehen ist, die Referenzbilder der Zielteile und Diagnosestandorte eines Objekts zu speichern, und wobei die Eingabeeinheit (700) dafür vorgesehen ist, eine Auswahl des Zielteils und eines Diagnosestandorts von dem Benutzer zu empfangen.

5. Ultraschallbildgebungsvorrichtung (100) nach Anspruch 1, wobei die Detektiereinheit wenigstens einen eines Winkelgeschwindigkeitssensors, eines magnetischen Sensors, eines Beschleunigungssensors, eines Schwerkraftsensors und eines Gyrosensors aufweist.

6. Verfahren zum Steuern der Ultraschallbildgebungsvorrichtung (100) nach Anspruch 1, welches Folgendes aufweist:
Erzeugen eines dreidimensionalen Bildgebungssignals eines Objekts;
Anzeigen eines Diagnosebilds in dem ersten Abschnitt basierend auf dem Bildgebungssignal;
Anzeigen einer Vielzahl von Referenzbildern ähnlich zu dem Diagnosebild in dem dritten Abschnitt vor dem Synchronisieren;
Empfangen einer Auswahl von einem Bild als das ausgewählte Referenzbild unter der Vielzahl von Referenzbildern von einem Benutzer;
Synchronisieren des Diagnosebilds mit dem ausgewählten Referenzbild entsprechend dem Diagnosebild und Festlegen der Position und des Standorts der Ultraschallsonde zu der Zeit der Synchronisierung als die Referenzposition und Referenzstandort der Ultraschallsonde; und
Anzeigen des Bildindikators, der das Zielteil des Objekts und die Richtung des Ultraschallsondenstrahls in dem zweiten Abschnitt kennzeichnet,
Detektieren einer relativen Position der Ultraschallsonde (200) relativ zu der Referenzposition und eines relativen Standorts der Ultraschallsonde (200) relativ zu dem Referenzstandort; und
Bewegen des Bildindikators gemäß der relativen Position und dem relativen Standort.

## Revendications

1. Appareil d'imagerie ultrasonique (100) comprenant :
une sonde ultrasonique (200) configurée pour générer un signal d'imagerie tridimensionnelle d'un objet en transmettant un faisceau ultrasonique, dans lequel la sonde ultrasonique (200) comprend une unité de détection configurée pour détecter une position relative de la sonde ultrasonique (200) par rapport à une position de référence et un emplacement relatif de la sonde ultrasonique (200) par rapport à un emplacement de référence ;
une unité d'affichage (600) configurée pour afficher une image de diagnostic dans une première section sur la base du signal d'imagerie, et afficher un indicateur d'image indiquant une partie cible de
l'objet dans une seconde section ; et
une unité de commande (400) configurée pour synchroniser l'image de diagnostic avec une image de référence sélectionnée correspondant à l'image de diagnostic et pour définir la position et l'emplacement de la sonde ultrasonique au moment de la synchronisation en tant que position et emplacement de référence de la sonde ultrasonique,
dans lequel l'image de référence comprend une image en coupe transversale d'une image de volume tridimensionnelle ;
dans lequel l'indicateur d'image comprend un marqueur d'organe cible qui est configuré pour afficher la partie cible en tant qu'image de volume tridimensionnelle identique à l'image de volume tridimensionnelle de l'image de référence et pour afficher un marqueur de direction de faisceau indiquant la direction du faisceau de sonde ultrasonique en trois dimensions :
et
dans lequel l'unité d'affichage est configurée pour afficher une pluralité d'images de référence similaires à l'image de diagnostic dans une troisième section, l'appareil d'imagerie ultrasonique comprend en outre une unité d'entrée (700) configurée pour recevoir une sélection d'une image en tant qu'image de référence sélectionnée parmi la pluralité d'images de référence provenant d'un utilisateur, et l'unité de commande (400) est configurée pour déplacer l'indicateur d'image en fonction de la position relative et de l'emplacement relatif.

2. Appareil d'imagerie ultrasonique (100) selon la revendication 1, dans lequel
l'indicateur d'image comprend en outre un marqueur d'axe corporel indiquant un emplacement anatomique de l'objet.

3. Appareil d'imagerie ultrasonique (100) selon la revendication 1, l'unité d'entrée (700) est en outre configurée pour recevoir une sélection de la partie cible et un emplacement de diagnostic d'un utilisateur,
dans lequel l'unité d'affichage (600) est en outre configurée pour afficher le marqueur d'organe cible dans la seconde section en fonction de la sélection de la partie cible et de l'emplacement de diagnostic.

4. Appareil d'imagerie ultrasonique (100) selon la revendication 1, comprenant en outre :
une unité de stockage (500) configurée pour stocker les images de référence des parties cibles
et des emplacements de diagnostic d'un objet, et l'unité d'entrée (700) est configurée pour recevoir une
sélection de la partie cible et un emplacement de diagnostic de l'utilisateur.

5. Appareil d'imagerie ultrasonique (100) selon la revendication 1,
dans lequel
l'unité de détection comprend au moins l'un d'un capteur de vitesse angulaire, d'un capteur magnétique, d'un capteur d'accélération, d'un capteur de gravité et d'un capteur gyroscopique.

6. Procédé de commande d'un appareil d'imagerie ultrasonique (100) selon la revendication 1 comprenant :
la génération d'un signal d'imagerie tridimensionnelle d'un objet ;
l'affichage d'une image de diagnostic dans la première section sur la base du signal d'imagerie ;
l'affichage d'une pluralité d'images de référence similaires à l'image de diagnostic dans la troisième
section avant la synchronisation ;
la réception d'une sélection d'une image en tant qu'image de référence sélectionnée parmi la pluralité d'images de référence provenant d'un utilisateur ;
la synchronisation de l'image de diagnostic avec
l'image de référence sélectionnée correspondant à
l'image de diagnostic et la définition de la position et de l'emplacement de la sonde ultrasonique au moment de la synchronisation en tant que position et emplacement de référence de la sonde ultrasonique ;
et
l'affichage de l'indicateur d'image indiquant la partie cible
de l'objet et la direction
du faisceau de sonde ultrasonique dans la seconde section,
la détection d'une position relative de la sonde ultrasonique (200) par rapport à la position de référence et un emplacement relatif de la sonde ultrasonique (200) par rapport à l'emplacement de référence ; et
le déplacement de l'indicateur d'image en fonction de la position relative et de l'emplacement relatif.
